# EUROPEAN PATENT APPLICATION

(11) **EP 0 668 056 A1**
(43) Date of publication of application: **23.08.1995**
(21) Application number: 95301068.3
(22) Date of filing: 20.02.1995
(51) Int. Cl.: A61B 17/04, A61B 17/06

(54) **Suture probe**

(30) Priority: 22.02.1994 US 200772
(71) Applicant: SMITH & NEPHEW DYONICS INC, Andover, Massachusetts 01810 (US)
(72) Inventor: Kennedy, John E., Lowell, Massachusetts 01852 (US); Torrie, Paul A., Marblehead, Massachusetts 01945 (US); Ek, Steven, Bolton, Massachusetts 01740 (US)
(74) Representative: Gilholm, Stephen Philip

(57) **Abstract**

A probe (10) for inserting suture (20) in a body comprises an elongated shaft (12) having an opening (18) therein adjacent to a tissue puncturing tip (16) for receiving an end (19) of the suture (20). Other portions of the suture (20) are disposed outside of the shaft (12). A clamp (22) releasably secures the end (19) of the suture (20) within the opening (18). In a preferred embodiment, the opening (18) is oriented at an acute angle (17) to the shaft (12), and the clamp (22) includes a rod (30) disposed for axial movement within the shaft (12) and having a distal end arranged to intercept the opening (18). An actuator (24) mounted to a handle (14) of the probe (10) engages a proximal end of the rod (30) to selectively move the rod (30) axially between a first position in which the distal end of the rod (30) clamps the end (19) of the suture (20) within the opening (18), and a second position in which the rod (30) releases the suture (20) for removal from the opening (18).

## Description

This invention relates to probes for inserting suture into body tissue, and in particular to suture probes for use in arthroscopy.

During arthroscopy, suture typically is passed into tissue through a puncture passage created by an elongated probe that carries the suture. The probe is advanced into the body through a cannula. One type of probe resembles a hypodermic needle that carries the suture within it. After puncturing, the suture is advanced through and out of the tip of the needle by thumbwheel-type actuator on the handle of the probe.

Other suture probes carry the suture externally to the probe. One such probe includes an open eyelet in its tip through which the suture is looped. The suture thus passes from the probe handle along one side of the probe, through the eyelet, and is doubled back along the opposite side of the probe to the handle. Using an actuator mounted on the handle, the user retracts the eyelet into a sleeve to retain the suture while the probe is passed through tissue. After puncturing, the eyelet is advanced, and the suture loop is pulled out of the eyelet.

One general aspect of this invention is a suture probe equipped with a clamp for releasably securing an end of the suture within an opening provided in the shaft of the probe adjacent to a tissue-puncturing tip, with other portions of the suture being disposed outside of the shaft.

Securing the end of the suture within the shaft with an internal clamp significantly reduces the size of the shaft-suture combination, thereby greatly easing the task of inserting the probe and suture through body tissue. Nothing need be slipped over the shaft to hold the suture in place, nor must the suture be doubled back along the length of the probe during tissue puncturing. Moreover, only the clamped end of the suture is within the shaft. The remainder of the suture lies outside of the shaft and accompanies the tip of the probe through the tissue. Thus, the end of the suture opposite the clamped end is free for grasping during suturing. That is, the suture need not be fed out of the tip of the probe to give the user sufficient suture length to work with.

Preferred embodiments include the following features.

The opening is a bore oriented at an acute angle (such as within the range of 15° and 25°, and preferably 20°) with respect to the shaft. The more acute the angle, the smaller the profile of the shaft and suture becomes. The bore is configured to block the end of the suture from passing completely through the shaft. In one embodiment, the bore has a closed end within the shaft. In another configuration in which the bore passes completely through the shaft, at least a portion of the bore has a diameter smaller than that of the suture.

The clamp includes a rod disposed within the shaft for urging the end of the suture against a stationary portion of the shaft. The rod is disposed within a passage in the shaft that intercepts the opening, and an actuator is operatively coupled to the rod to move the rod axially within the passage to selectively clamp and release the end of the suture. The actuator is mounted to a handle that supports the shaft and engages the proximal end of the rod.

In one embodiment, the actuator is mounted for axial movement with respect to the handle so that axial motion of the actuator causes the rod to move axially within the passage between a first position in which the rod clamps the end of the suture within the opening, and a second position in which the rod releases the suture for removal from the opening. A spring urges the rod into the first position. In another embodiment, the actuator is rotatably mounted to the handle and includes a mechanism for converting the rotation of to actuator into axial motion of the rod between the first and second positions.

The rod is a metal wire. Examples of suitable metal include stainless steel and Nitinol (an acronym that stands for an alloy of nickel and titanium developed at the Naval Ordnance Laboratory). A plastic element may be disposed on a distal end of the wire for engaging the end of the suture. The plastic element is sufficiently soft to avoid damaging the suture during clamping. The shaft may be straight or curved.

Other features and advantages of the invention will become apparent from the following detailed description, and from the claims.

Fig. 1 shows a suture probe.

Fig. 2 illustrates the tip of the suture probe.

Figs. 3 and 4 are cross-sectional views of the tip useful in understanding how the suture probe clamps and releases suture within the tip.

Fig. 5 is an exploded view of the handle of the suture probe.

Fig. 6 shows an alternative embodiment of the handle.

Fig. 7 shows a curved suture probe.

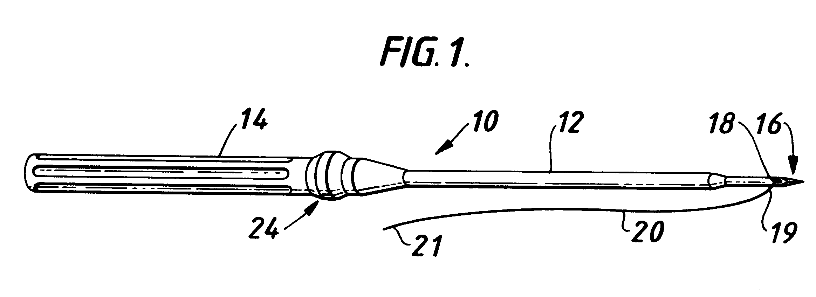

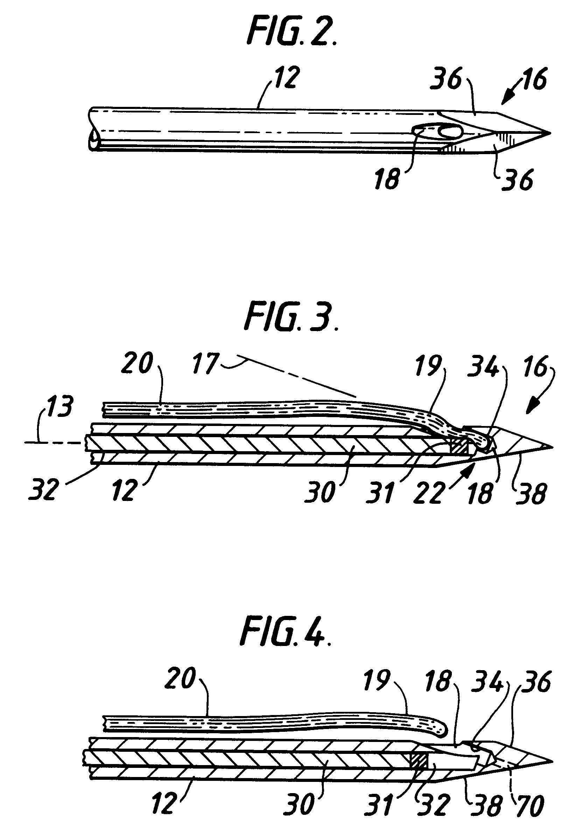

Referring to Figs. 1 and 2, suture probe 10 includes an elongated shaft 12 which extends distally from a handle 14. The tip 16 of shaft 12 is sharpened so that shaft 12 serves as a trocar for puncturing body tissue. An opening 18 is disposed in shaft 12 proximally adjacent tip 16 for receiving an end 19 of suture 20. As described in detail below, a clamp 22 (shown in Figs. 3 and 4) operated by an actuator 24 mounted on handle 14 releasably secures end 19 of suture 20 within opening 18. The remainder of suture 20, including opposite end 21, extends proximally along the outside of shaft 12.

With end 19 of suture 20 clamped within opening 18, shaft 12 is inserted into the tissue to create an opening through which the length of suture 20 also passes. As described below, clamp 22 is then released to allow suture end 19 to be removed from opening 18 so that suturing can proceed. Because end 19 of suture 20 is held in place within shaft 12 near tip 16 (rather than being looped through an eyelet or the like near tip 16), suture 20 need not be doubled back along the length of shaft 12 during insertion. This reduces the total size of the shaft-suture combination, thereby easing the task of advancing shaft 12 and suture 20 through the tissue.

Referring to Fig. 3, clamp 22 includes a rod 30 that is moved axially within a passage 32 of shaft 12 by actuator 24 to urge suture end 19 against an interior surface 34 of shaft 12. Rod 30 is a wire made from any suitable material, such as stainless steel or Nitinol. A plastic element 31 may be disposed on the distal end of rod 30 to reduce the risk of damage to suture end 19 during clamping. Passage 30 is coaxial with the axis of elongation 13 of shaft 12 and is approximately the same diameter as rod 32 (a gap between them is enlarged in the drawings).

Opening 18 is a cylindrical bore drilled into shaft 12. The axis 17 of opening 18 defines an acute angle with axis 13. Preferably, this angle is between 15° and 25°, and more preferably is 20°. As shown in Fig. 2, opening 18 is disposed between the proximal ends of a pair of beveled surfaces 36 of tip 16. A third beveled surface 38 (seen in Fig. 3) meets surfaces 36 to form sharp tip 16.

Fig. 3 shows clamp 22 in position to secure suture end 19 within opening 18; clamp 22 is shown released in Fig. 4. In operation, with rod 30 in the released position, the user inserts end 19 of suture 20 into opening 18 and uses actuator 24 (as discussed below) to slide rod 30 axially toward tip 16. The distal end of rod 30 engages suture end 19 and urges it toward surface 34 of opening 18. When rod 30 is in its full forward position --shown in Fig. 3-- rod 30 compresses suture end 19 against surface 34, thereby capturing suture 20 in place within opening 18.

Probe 10 is then inserted into the body through a cannula (not shown) with suture 20 trailing from tip 16 alongside the outer surface of shaft 12. Tip 16 is pushed through the tissue, and suture 20 accompanies shaft 12 as it is advanced through the hole created by tip 16. When shaft 12 has been advanced sufficiently, actuator 24 is used to release clamp 22 by sliding rod 30 axially away from tip 16 (Fig. 4). Next, end 19 of suture 20 is removed from opening 18. At this point, suture 20 extends through the tissue, with end 19 and end 21 (Fig. 1) of suture 20 located on opposite sides of the hole made by probe 10.

Suturing then proceeds in any one of a number of ways, as desired by the user. One option is to withdraw shaft 12 from the tissue hole and clamp the proximal end 21 of suture 20 within opening 18 (using the procedure discussed above). Then, shaft 12 is inserted into the tissue adjacent the first-formed hole, and end 21 is removed from opening 18 as discussed above. At this point, suture 20 forms a loop through two holes formed in the tissue by probe 10.

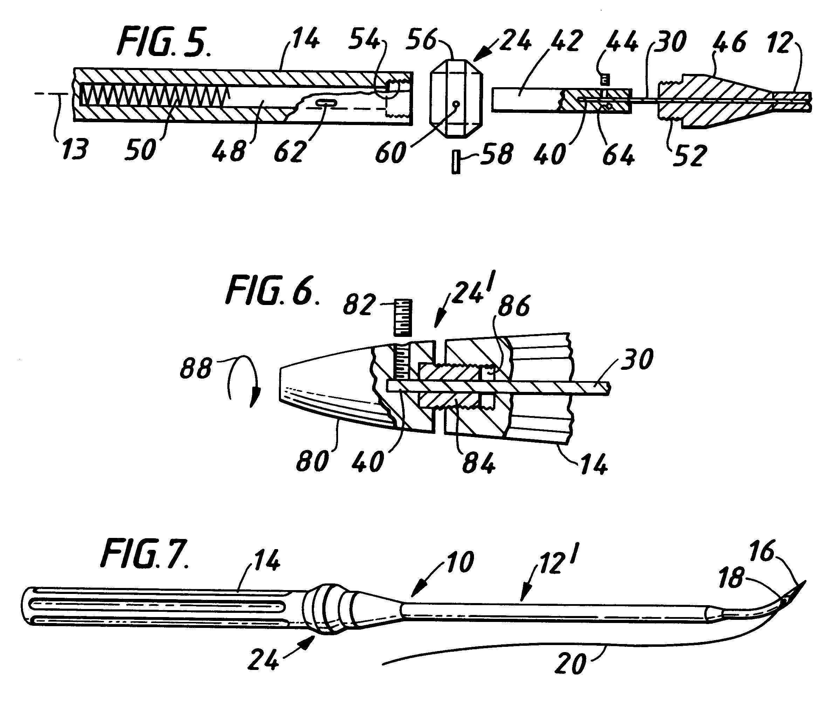

Referring Fig. 5, actuator 24 is a spring-loaded mechanism mounted on handle 14. The proximal end 40 of rod 30 is inserted into a cylindrical plug 42 and is secured therein by a set screw 44. Rod 30 extends distally from plug 42 into shaft 12 slidably through end cap 46 of handle 14. (Shaft 12 is attached to cap 46 by any suitable means, such as ultrasonic welding.)

Plug 42 fits within a cavity 48 in handle 14. A spring 50 is disposed between plug 42 and the base of cavity 48 for purposes to be described. A threaded extension 52 of end cap 46 mates with an enlarged, threaded opening 54 in handle 14 to secure plug 42 within cavity 48. Actuator ring 56 surrounds handle 14 and is linked to plug 42 by a pin 58 that passes through a hole 60 in ring 56, an axially elongated slot 62 in handle 14, and a hole 64 in plug 42. Pin 58 is press fit within holes 60 and 64.

Spring 50 urges plug 42, and thus rod 30, axially forward toward tip 16 (Fig. 3). Put another way, spring 50 biases clamp 22 in the closed position shown in Fig. 3. To open clamp 22, the user slides actuator ring 56 axially away from tip 16, overcoming the force of spring 50. This causes pin 58 to pull plug 42 (and thus rod 30) axially away from tip 16 through shaft 12 and cap 46, thereby opening clamp 22 (Fig. 4). Slot 62 limits the extent to which rod 30 travels axially.

This configuration affords easy, two handed operation of suture probe 10. For example, the user holds handle 14 in, say, the right hand and uses the fingers of this hand to slide actuator ring 56 proximally over handle 14 to open clamp 22. The left hand is used to insert suture end 19 (Fig. 4) into opening 18, and then actuator ring 56 is released, returning clamp 22 to the closed position (Fig. 3) to secure end 19 of suture 20 in place within opening 18.

Other embodiments are within the scope of the claims.

For example, while opening 18 has been shown closed within shaft 12, an extension 70 of opening 18 (shown in phantom in Fig. 4) may pass completely through shaft 12 to, for example, facilitate cleaning. Extension 70 should be smaller in diameter than the remainder of opening 18 to block suture 20 from passing completely through shaft 12.

The angle between opening 18 and axis 13 may be varied as desired. Decreasing the acuteness of the angle increases the length of opening 18, and thus may enhance clamping strength. In addition, the profile of shaft 12 and suture 20 adjacent to tip 16 is reduced as the angle of opening 18 is decreased, which further eases insertion through tissue. But as the angle is decreased, the spacing between proximal end of opening 18 and tip 16 increases. It is often desirable to keep this spacing as small as possible, so that end 19 of suture 20 closely follows tip 16.

While shaft 12 has been shown as cylindrical, other configurations are possible. For example, a channel may be formed in shaft 12 along its length for receiving the length of suture 20. This would reduce the overall size of the shaft-suture combination, while maintaining suture 20 external to shaft 12 proximally of opening 18. Among other advantages, end 21 is kept free for grasping by the user, without having to draw the length of suture 20 through shaft 12.

Alternative materials may be used. For example, rod 30 may be made from a material other than metal, so long as rod 30 is sufficiently stiff to clamp suture 20 within opening 22. Element 31 (Fig. 3) need not be plastic. A soft metal (such as bronze) may be used instead.

Referring to Fig. 6, other mechanisms for actuator 24 are possible. Actuator 24' includes a knob 80 that is rotatable with respect to handle 14 to move rod 30 between the clamped (Fig. 3) and released (Fig. 4) positions. The proximal end 40 of rod 30 extends through handle 14 and is secured within knob 80 by a set screw 82. A threaded extension 84 of knob 80 (through which rod 30 also passes) is received by a threaded cavity 86 at the base of handle 14.

Fig. 6 shows actuator 24' in a position that corresponds to the released position of clamp 22 (Fig. 4). To close clamp 22 against suture 20 (Fig. 3), the user rotates knob 80 clockwise (as shown by arrow 88) with respect to 14. As extension 84 advances within cavity 86, rod 30 is moved axially within handle 14 and shaft 12 to the clamped position shown in Fig. 4. Knob 80 is rotated counterclockwise to release clamp 22.

Referring to Fig. 7, suture probe 10 may alternatively have a curved shaft 12' (rather than the straight shaft 12 shown in Fig. 1). The degree of curvature is sufficiently small that shaft 12' can be inserted through a cannula, though this is not a requirement.

Other shapes are also possible. For example, shaft 12 may be bent at a 90° angle in any direction (e.g., left, right, up, or down) with respect to handle 14. Shaft 12 may alternatively be "U" shaped (so that tip 16 faces handle 14). In still another configuration, shaft 12 may be helical or S-shaped. In all of these configurations, the amount of curvature and the flexibility of rod 30 should be selected so that rod 30 can be moved axially within shaft 12 to clamp and release suture 20.

## Claims

1. A probe for inserting suture in a body, comprising
an elongated shaft including a distal tip for puncturing body tissue, said shaft having an opening therein adjacent said tip for receiving an end of the suture, with other portions of said suture being disposed outside of said shaft, and
a clamp for releasably securing said end of said suture within said opening.

2. The probe of claim 1 wherein said opening includes a bore oriented at an acute angle with respect to said elongated shaft.

3. The probe of claim 2 wherein said acute angle is within a range of 15° and 25°.

4. The probe of claim 2 wherein said bore is configured to block said end of said suture from passing completely through said shaft.

5. The probe of claim 4 wherein said bore has a closed end within said shaft.

6. The probe of claim 4 wherein said bore passes completely through said shaft, at least a portion of said bore having a diameter smaller than a diameter of said suture to block said end of said suture from passing completely through said shaft.

7. The probe of claim 1 wherein said clamp includes a rod disposed within said shaft for urging said end of said suture against a stationary portion of said shaft.

8. The probe of claim 7 further comprising an actuator for moving said rod to selectively clamp and release said end of said suture.

9. The probe of claim 8 wherein said rod is disposed within a passage in said shaft that intercepts said opening, said actuator being operatively coupled to said rod to move said rod axially within said passage.

10. The probe of claim 9 further comprising a handle to which said shaft is mounted, said actuator being mounted to said handle and engaging a proximal end of said rod.

11. The probe of claim 10 wherein said actuator is mounted for axial movement with respect to said handle so that axial motion of said actuator causes said rod to move axially within said passage between a first position in which said rod clamps said end of said suture within said opening and a second position in which said rod releases said suture for removal from said opening.

12. The probe of claim 11 further comprising a spring for urging said rod into said first position.

13. The probe of claim 10 wherein said actuator is rotatably mounted to said handle and includes means for causing rotation of said actuator to move said rod axially within said passage between a first position in which said rod clamps said end of said suture within said opening and a second position in which said rod releases said suture for removal from said opening.

14. The probe of claim 7 wherein said rod comprises a metal wire.

15. The probe of claim 14 further comprising a plastic element disposed on a distal end of said wire for engaging said end of said suture.

16. The probe of claim 1 wherein said shaft is straight.

17. The probe of claim 1 wherein said shaft is curved.

18. A probe for inserting suture in a body, comprising
an elongated shaft extending from a handle and including a distal tip for puncturing body tissue,
said shaft having an opening therein adjacent said tip and
oriented at an acute angle to said shaft for receiving an end of the suture,
a rod disposed for axial movement within said shaft and having a distal end arranged to intercept said opening,
an actuator mounted to said handle for engaging a proximal end of said rod to selectively move said rod axially within said shaft between a first position in which said distal end of said rod clamps said end of said suture within said opening, and a second position in which said rod releases said suture for removal from said opening.
